Europäisches Patentamt

European Patent Office

Office européen des brevets

(11) Publication number: **0 324 478**
**A1**

## EUROPEAN PATENT APPLICATION

(21) Application number: 89100487.1

(22) Date of filing: 12.01.89

(51) Int. Cl.⁴: **C07C 19/08 , C07C 17/00**

(30) Priority: 15.01.88 IT 1907788

(43) Date of publication of application:
19.07.89 Bulletin 89/29

(84) Designated Contracting States:
**BE DE ES FR GB NL SE**

(71) Applicant: **AUSIMONT S.r.l.**
**31, Foro Buonaparte**
**I-20121 Milano(IT)**

(72) Inventor: **Gervasutti, Claudio**
**2/4 via P. Sarpi**
**I-30172 Mestre Venezia(IT)**

(74) Representative: **Barz, Peter, Dr. et al**
**Patentanwälte Dipl.-Ing. G. Dannenberg Dr.**
**P. Weinhold, Dr. D. Gudel Dipl.-Ing. S.**
**Schubert, Dr. P. Barz Siegfriedstrasse 8**
**D-8000 München 40(DE)**

(54) Process for preparing 1,2-difluoroethane and 1,1,2-trifluoroethane.

(57) A process for preparing 1,2-difluoroethane or 1,1,2-trifluoroethane by hydrogenation of 1,2-dichloro-difluoroethylene or chlorotrifluoroethylene in the presence of a hydrogenation catalyst, said process being conducted in the gas phase at a temperature ranging from 100 to 220°C, the molar ratio of $H_2$ to olefin being at least 3.

EP 0 324 478 A1

## PROCESS FOR PREPARING 1,2-DIFLUOROETHANE AND 1,1,2-TRIFLUOROETHANE

The present invention relates to a process for preparing partially hydrogenated fluorinated hydrocarbons. Particularly, it relates to the preparation of 1,2-difluoroethane and of 1,1,2-trifluoroethane by catalytic hydrogenation of 1,2-dichloro-difluoroethylene and chlorotrifluoroethylene, respectively.

1.2-difluoroethane $CH_2F-CH_2F$ is a well-known fluorinated hydrocarbon which is utilizable, in admixture with other hydrocarbons, as fluid for the Rankine cycle, as cooling medium (US-A-4 055 049) or as starting material for the preparation of fully substituted brominated derivatives.

1,2-difluoroethane and 1,1,2-trifluoroethane can also be used as components of propellant mixtures for aerosols.

In fact, although the assumption that a possible accumulation of fluorinated hydrocarbons may lead to a degradation of the ozone in the stratosphere is still to be proved, it conversely seems certain that the hydrogen-containing fluorocarbons do not cause any problems in this respect.

The prior art does not disclose any specific method for preparing 1,2-difluoroethane which can be easily practised on an industrial scale or which is capable of providing this product in high yields and with high selectivities. Conversely, the prior art describes general methods for the treatment of various substituted ethanes from which it is possible to obtain a mixture of products in which 1,2-difluoroethane may also be present as a by-product.

These general methods include the direct fluorination of $CH_3-CH_2F$, with $F_2$ (Cadmau P., Kirk A.W., Trotman-Dickenson A.F., J. Chem. Soc., Faraday Trans. 1), the electrochemical fluorination of ethane at $100\,°C$ (Fox H.M., Ruehlen F.N., Childs W.V., J. Electrochem. Soc. 1971, 118(7), 1246-9) and the fluorination of ethane with potassium tetrafluorocobaltate ($KCoF_4$) and $CoF_3$ (Burdon J., Knights J., Parson I., Tatlow J. - Tetrahedron 1976, 32(9), 1041-3).

All the above processes are very difficult to carry out on an industrial scale, either in view of the high costs of the equipment involved due to the presence of $F_2$ or due to the use of catalysts such as, for example, $CoF_3$, which requires very sophisticated apparatus for its regeneration with $F_2$, said regeneration being necessary because of the reduction of the catalyst during the reaction.

FR-A-86 08389, in the name of applicant, describes a process for preparing halogenated olefins by catalytic hydrogenation of 1,2-dichloro-difluoroethylene at temperatures higher than $100\,°C$, in particular, from 200 to $600\,°C$, preferably from 300 to $400\,°C$.

According to the teaching of said document, the hydrogenolysis of 1,2-dichloro-difluoroethylene in the presence of palladium as catalyst involves the partial or complete substitution of chlorine, while the ethylenic unsaturation of the starting compound is left unaffected. In all the examples described in the above FR-A-86 08389, a temperature of 300 or $350\,°C$ was used.

High conversions, even higher than 90%, were obtained and, as by-products, a mixture of $CH_2F-CH_2F$, CHClF-CHClF and $CH_2=CHF$ in an amount not exceeding 20% was present.

According to said document, the molar ratio of hydrogen to olefin can vary over a wide range, i.e., from 0.5 to 10, preferably from 3 to 5. The lower the reaction temperature, the higher is the necessary ratio of hydrogen to olefin, namely, higher than 2/1, in order to obtain high yields of the olefins. If the temperatures are equal, high yields of the olefins are obtained when high molar ratios (higher than 2/1) are employed.

From applicant's older patent application EP-A-253 410 it is known how to prepare fluorinated or chlorinated olefins, in particular fluoro- and chloroethylenes such as, for example, CHF = CHF and CFCl = CFH, starting from chlorofluoroethanes and hydrogen in the presence of hydrogenation catalysts.

The reaction temperatures range from 150 to $600\,°C$, preferably from 200 to $400\,°C$.

According to said application, experimental tests have proved that it is necessary to operate at high temperatures and with high ratios of hydrogen to chlorofluoroethane in order to achieve high conversions.

However, the conversion is never 100%, its maximum value being about 85%, which, in the course of time, results in a deactivation of the catalyst and, consequently, significant drawbacks for a commercial-scale process.

Thus, it is an object of the present invention to provide a process for preparing 1,2-difluoroethane in high yields and with high conversions, free of the above drawbacks, particularly, the deactivation of the catalyst after operation over extended periods of time.

A further object of the invention is the provision of a process for selectively preparing 1,1,2-trifluoroethane which comprises reacting chlorotrifluoroethylene (CTFE) with hydrogen in the presence of a hydrogenation catalyst, said process being carried out in the gas phase at temperatures ranging from 100 to $220\,°C$.

It has, surprisingly, been found that, if a particular combination of specific process parameters is used,

2

it is possible to obtain, in high yields and with high conversions, 1,2-difluoroethane and 1,1,2-trifluoroethane.

The reaction temperature is the most critical parameter of the process of the present invention, in combination with other parameters, such as the contact time between $H_2$ and olefin, and the molar ratio $H_2$/reagents, for achieving a high conversion of the reagents and high yields of products.

It has, surprisingly, been found that, if the hydrogenolysis is conducted at temperatures ranging from 100 to 220°C with contact times of at least 30 seconds and molar ratios $H_2$/reagents which range from 3 to 10, the starting compounds lose their ethylenic unsaturation and 1,2-difluoroethane and 1,1,2-trifluoroethane are obtained in high yields and with high conversions, and the catalyst can be used for very long periods of time without exhibiting deactivation.

Preferably, the parameter combination is as follows: A temperature of from 120 to 180°C, a contact time of from 30 to 45 seconds and molar ratios of hydrogen to starting olefin of from 4:1 to 6:1 in the case of 1,2-dichloro-difluoroethylene and of from 3.5:1 to 5:1 for chlorotrifluoroethylene.

At temperatures higher than 220°C, the yield of 1,2-difluoroethane and 1,1,2-trifluoroethane drastically decreases while, at temperatures lower than 100°C, the conversion of 1,2-dichloro-difluoroethylene and chlorotrifluoroethylene is rather low and of no interest from an industrial point of view.

1,2-dichloro-difluoroethylene and CTFE, utilized as starting materials in the process of the present invention, are commercial products which can easily be prepared by dechlorination of 1,2-difluorotetrachloroethane and of $CCl_2F$-$CClF_2$.

According to the process of the present invention, a gaseous mixture of hydrogen and olefin in a molar ratio ranging from 3:1 to 10:1 is caused to flow through the reaction zone so as to bring the mixture into intimate contact with the catalyst (maintained at 100 to 220°C) for 30 to 60 seconds. The products may be recovered from the effluent gases.

The catalytic hydrogenation process of the invention can be conducted at atmospheric pressure as well as at superatmospheric pressure. Pressures of up to 15 bar can be employed.

One of the most important features of the process of the present invention is, as already pointed out, the reaction temperature. This parameter must, however, be seen in connection with the contact times and the reagent ratios.

In order to obtain high conversions and high yields, it is preferable to choose a contact time of from 30 to 60 seconds. In fact, contact times of less than 30 seconds result in a strong decrease in the conversion of the starting compounds, while contact times of more than 1 minute are useless and do not lead to a substantial increase of conversion and yield.

The hydrogen/olefin molar ratio should range from 3 to 10, and preferably from 4 to 6 for 1,2-dichloro-difluoroethylene and from 3.5 to 5 for chlorotrifluoroethylene. In fact, if the molar ratios are lower than 3, a drastic reduction in the conversion of the starting materials occurs while, if the hydrogen is employed in amounts that are too high, the unreacted excess hydrogen cannot be recovered unless complicated procedures are resorted to.

The catalytic bed over which the gaseous reagent mixture is made to flow comprises a hydrogenation catalyst which comprises a transition metal such as palladium, platinum, nickel, chrome, copper, either as such or, preferably, supported on an inert material such as, for example, carbon, alumina, $BaSO_4$, etc. at concentrations of from 0.1 to 5% by weight.

The catalyst preferably consists of palladium, optionally supported on carbon, at concentrations ranging from 0.1 to 5% by weight. Small amounts, up to 10% by weight, of other metal catalysts (for example copper, nickel, chrome) may additionally be present.

Hydrogen can be employed either in pure state or diluted with an inert gas such as, for example, nitrogen, helium, argon etc.

The hydrogenation process is usually conducted in tubular reactors made of materials which are inert towards the reagents, the products and the by-products of the reaction, such as nickel, Inconel, stainless steel, etc.

The reaction products and the unconverted reagents can be recovered and isolated by conventional methods. For example, the gaseous effluent from the reactor may first be passed through an aqueous solution containing alkali metal hydroxide in order to remove the hydrochloric acid, whereafter it may be dried over $CaSO_4$ and, finally, condensed in a trap cooled with dry ice and methanol.

The following examples illustrate the present invention without being a limitation thereof.

In the examples parts and percentages are by weight unless otherwise specified.

EXAMPLE 1

3

Into a cylindrical reactor made of AISI 316, having an internal diameter of 5.4 cm and a length of 70 cm and containing 480 ml of granules of activated carbon with a palladium content of 1% by weight and thermoregulated at 130°C, there were introduced, at atmospheric pressure, 1.28 moles/h of a mixture, preheated to 90°C, of hydrogen and 1,2-dichloro-difluoroethylene in a molar ratio $H_2$ $C_2Cl_2F_2$ of 5:1. The contact time was 40 seconds.

The gaseous stream leaving the reactor was washed with an aqueous 5% NaOH solution to remove hydrochloric acid, whereafter it was dried with calcium sulphate and condensed in a trap cooled to -70°C with dry ice and methanol.

After the washing step with the NaOH solution, the gases leaving the reactor exhibited the following composition (based on gas-chromatographic analysis):

| | |
|---|---|
| 1,2-difluoroethane ($CH_2F$-$CH_2F$) | 90% |
| 1,2-difluoroethylene (CHF = CHF) | 5% |
| 1-chloro-2-fluoroethane ($CH_2Cl$-$CH_2F$) | 3% |
| unreacted 1,2-dichloro-difluoroethylene (CCIF = CCIF) | absent |

The balance of 100% consisted of by-products such as CHF = CCIF, $CH_2$ = CHF.

## EXAMPLE 2

Into the reactor described in example 1, thermoregulated at 200°C, a hydrogen/1,2-dichloro-difluoroethylene mixture (molar ratio 4:1) was introduced. The contact time was 35 seconds. The catalyst used and the other procedures employed were the same as in example 1.

The gases, after having left the reactor and after an alkaline washing, were subjected to gas-chromatographic analysis. The weight percentages determined were as follows:

| | |
|---|---|
| 1,2-difluoroethane | 60% |
| 1,2-difluoroethylene | 20% |
| 1-chloro-1,2-difluoroethylene | 7% |
| 1-chloro-2-fluoroethane | 8%. |

## EXAMPLE 3

Into a cylindrical nickel reactor having an internal diameter of 2 cm and an actual volume of 160 ml, thermoregulated at 150°C and containing 100 ml of granules of activated carbon with a palladium content of 0.5%, 0.22 moles/h of a mixture of hydrogen and 1,2-dichloro-difluoroethylene in a molar ratio of 5:1 were fed.

The gas-chromatographic analysis of the gases leaving the reactor, after the washing step, revealed the following composition:

| | |
|---|---|
| 1,2-difluoroethane | 75% |
| 1,2-difluoroethylene | 12% |
| 1-chloro-1,2-difluoroethylene | 7% |
| 1-chloro-2-fluoroethane | 3%. |

## EXAMPLE 4 (comparative example with regard to example 3)

Into a cylindrical nickel reactor, thermoregulated at 150°C, having an internal diameter of 2 cm and an actual volume of 160 ml and containing 100 ml of granules of activated carbon with a palladium content of 1% by weight, a hydrogen/1,2-dichloro-difluoroethylene mixture (molar ratio of 4:1) was fed at a flow rate of 0.66 moles/hour. The contact time was 15 seconds.

The gas-chromatographic analysis of the effluent from the reactor, carried out after an aqueous washing step, showed the following results:

| | |
|---|---|
| 1,2-difluoroethane | 3% |
| 1,2-difluoroethylene | 6% |
| 1-chloro-1,2-difluoroethylene | 20% |
| unreacted 1,2-dichloro-difluoroethylene | 70%. |

EXAMPLE 5 (comparative example with regard to example 3)

Working under the conditions of example 4, to the same catalytic system, thermoregulated at 150° C, 0.22 moles/h of a mixture of hydrogen and 1,2-dichloro-difluoroethylene in a molar ratio $H_2/C_2Cl_2F_2$ of 2:1 were fed.

After an alkaline washing step, the organic effluent from the reactor was subjected to gas-chromatographic analysis which revealed the weight percentages indicated below. The contact time was 45 seconds.

| | |
|---|---|
| 1,2-difluoroethane | 2% |
| 1,2-difluoroethylene | 8% |
| 1-chloro-1,2-difluoroethylene | 15% |
| unreacted 1,2-dichloro-difluoroethylene | 75% |

EXAMPLE 6

To a cylindrical reactor made of AISI 316, having an internal diameter of 5 cm and a length of 50 cm and containing 230 ml of active carbon with a palladium content of 1% by weight, thermoregulated at 130° C, there were fed, at atmospheric pressure, 0.83 moles/h of a mixture, preheated at 70° C, of $H_2$ and 1-chlorotrifluoroethylene in a molar ratio $H_2/C_2ClF_3$ of 3.75:1. The contact time was 30 seconds.

The gases leaving the reactor, after an alkaline washing step, had the following composition as determined by gas-chromatographic analysis (in weight percent):

| | |
|---|---|
| 1,1,2-trifluoroethane | 96% |
| trifluoroethylene | 1% |
| unreacted 1-chlorotrifluoroethylene | 2%. |

Claims

1. Process for preparing 1,2-difluoroethane or 1,1,2-trifluoroethane, which comprises reacting 1,2-dichloro-difluoroethylene or chlorotrifluoroethylene, respectively, with hydrogen in the gas phase at temperatures ranging from 100 to 220° C, within contact times ranging from 30 to 60 seconds, at a molar ratio of $H_2$ to olefin of from 3 to 10, in the presence of a hydrogenation catalyst comprising a transition metal.

2. Process according to claim 1, wherein the temperature ranges from 120 to 180° C.

3. Process according to any one of claims 1 or 2, wherein the transition metal is supported on an inert material at a concentration ranging from 0.1 to 5% by weight.

4. Process according to any one of claims 1 to 3, wherein the hydrogenation catalyst is Pd.

5. Process according to claim 4, wherein Pd is present along with other metals such as Cu, Ni and Cu or Cr and Cu.

6. Process according to any one of claims 1 to 5, wherein the contact time of the reagent mixture and the catalytic bed ranges from 30 to 45 seconds.

7. Process according to any one of claims 1 to 6, wherein the $H_2$/olefin ratio ranges from 4:1 to 6:1 for 1,2-dichloro-difluoroethylene and from 3.5:1 to 5:1 for chlorotrifluoroethylene.

8. 1,2-Difluoroethane, prepared according to the process of one or more of claims 1 to 7.

9. 1,1,2-Trifluoroethane, prepared according to the process of one or more of claims 1 to 7.

European Patent Office

**EUROPEAN SEARCH REPORT**

EP 89100487.1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | EP - A1 - 0 053 657 (ALLIED COR-PORATION)<br><br>* Page 6, lines 1-12, especially lines 10-12; page 5, lines 24-30; table 2 *<br><br>-- | 1-4,9 | C 07 C 19/08<br><br>C 07 C 17/00 |
| D,A | FR - A1 - 2 583 039 (AUSIMONT)<br><br>* Claims; example 1 *<br><br>-- | 1-5,7, 8 | |
| D,P<br>A | EP - A1 - 0 253 410 (AUSIMONT)<br><br>* Claims; examples 7,14 *<br><br>-- | 1-5,7-9 | |
| A | GB - A - 1 578 933 (ICI)<br><br>* Claims 1,5,8,9-13; example 9 *<br><br>---- | 1,3,4, 6 | |
| | | | **TECHNICAL FIELDS SEARCHED (Int. Cl.4)**<br><br>C 07 C 19/00<br><br>C 07 C 17/00<br><br>C 07 C 21/00 |

The present search report has been drawn up for all claims

| Place of search | Date of completion of the search | Examiner |
|---|---|---|
| VIENNA | 23-03-1989 | KÖRBER |